# EUROPEAN PATENT SPECIFICATION

(11) **EP 0 872 231 B1**
(45) Date of publication and mention of the grant of the patent: **04.09.2002**
(21) Application number: 97830169.5
(22) Date of filing: 14.04.1997
(51) Int. Cl.: A61K 9/00, A61K 9/48

(54) **Pharmaceutical compositions comprising lactobacilli suitable for trans-mucosal administration**
Pharmazeutische Zusammensetzungen mit Laktobazillen zur transmucosalen Verabreichung
Compositions pharmaceutiques à base de lactobacilles pour l'administration transmucosale

(43) Date of publication of application: 21.10.1998
(73) Proprietor: DR. A. TOSI FARMACEUTICI S.R.L., I-28100 Novara (IT)
(72) Inventor: Tosi, Silvana, 28100 Novara (IT); Dondi, Giancarla, 28100 Novara (IT)
(74) Representative: Minoja, Fabrizio, Dr.

(56) References cited:
- EP-A- 0 353 581
- WO-A-84/04675
- US-A- 4 956 295

## Description

The present invention concerns pharmaceutical compositions comprising lactobacilli suitable for transmucosal administration.

The therapeutic use, in particular the use of lactobacilli, has been known for long time.

In particular, the use of lactobacilli in gynaecology for the treatment of the vaginal bacterial flora's disorders, has been described for example in FR M6836 and US 4 592 748.

The patent EP-B-353851 discloses a strain of Lactobacillus fermentum, deposited by the Institut Pasteur on 21.07.1988 at the number C.N.C.M. I-789, which is characterized by direct inhibitory activity towards patogen fungi, particularly against Candida albicans.

Said patent describes also pharmaceutical compositions in form of cream, vaginal ovules or capsules containing the Lactobacillus fermentum strain C.N.C.M. I-789 optionally associated to other Lactobacillus strains and to conventional excipients.

Among the vaginal administration forms, the soft gelatin capsules wherein the lyophilized microrganisms are suspended in an excipient like a medium-chain triglyceride mixture or other oleous excipient (Migliol^{(R)}) are generally preferred for their use practicity.

However an unsatisfying stability has been noted for the compositions described in EP-B-353 381, which are subjected to a two order of magnitude reduction in the bacterial charge just after few months at room temperature.

The clinical experiences carried out on the Lactobacillus fermentum I-789 have evidenced an optimal dose of at least one billion (10⁹) of living cells.

The study of a pharmaceutical form which allows the minimum number of the required cells to survive in the validity interval of the same preparation is thus necessary for the commercialization.

Now, it has been found that the use of the silica gel in particular ratios with respect to the weight of the lyophilized microorganism increases in a completely unexpected way the cell viability also for long periods, ranging up to 2-3 years. Such a stabilizing effect of the silica on the lactobacillus cell viability was not predictable on the basis of the state of the art which provides mostly the use of the silica in solid oral pharmaceutical formulations like tablets or granulates as an anti-adhesive agent.

The known de-humidifying and water-absorbent activities of the silica gel are not sufficient to explain the surprising stability improvement also in view of the fact that the lyophilized microrganisms should be already sufficiently protected from the water absorption by the gelatin envelopes and by the lipophylic excipient in which they are suspended.

Thus the invention refers to soft gelatin-vaginal capsules containing a therapeutically active amount of lyophilized Lactobacillus fermentum I-789, suspended in an appropriate vehicle, characterized in that it contains from 2 to 5% by weight of silica gel or precipitated silica, with respect to the weight of the lyophilizate.

The compositions of the invention optionally contain other known eubiotic bacterial strains, in particular those described in EP-B-353 581.

The percentage of silica gel is preferably comprised between 3 and 4% by weight and more preferably it is about 3,6% by weight with respect to the weight of lyophilized I-789.

Suitable suspending vehicles are represented by medium-chain semisynthetic triglycerides like Migliol^{(R)}. The gelatin envelopes, generally consisting of gelatin mixtures, dimethylpolysiloxane, glycerol and titanium dioxide, are commercially available. The capsules sold by Scherer under the trademark "Softigel^{(R)}" are particulary preferred.

The preparation procedure for the capsules of the invention is substantially of the conventional type and it is preferably carried out by performing all the operations under inert gas atmosphere, for example under nitrogen.

Thus the obtained capsules can be packaged into blisters or other packages, impermeable to the humidity, optionally comprising suitable drying materials.

In the following table the stabilities of the capsules which are the object of the invention -are reported, compared to the stabilities known from EP-B-353 581.

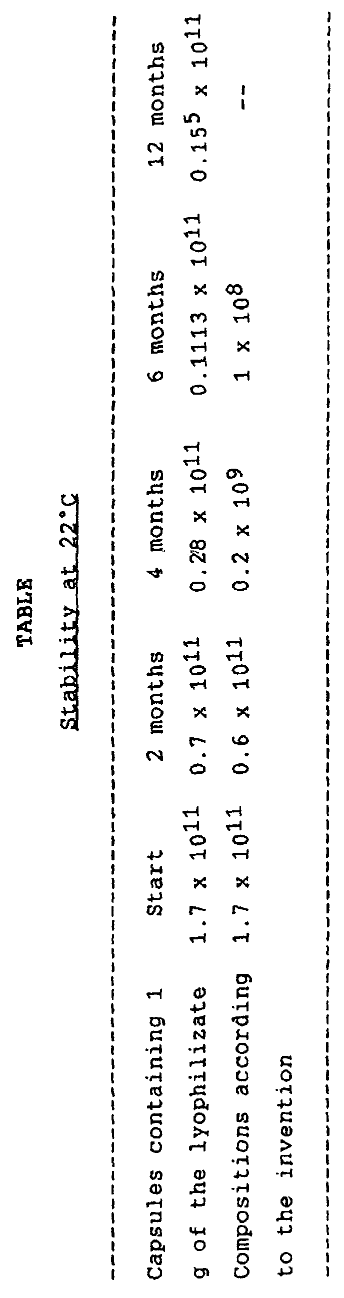

The data clearly demonstrate that the compositions of the invention mantain an high bacterial charge at least up to 12 months, while the known compositions show a non-acceptable bacterial charge decrease just after only 4 months at 22°C. After 6 months, the count value is already under the prescribed therapeutic amount.

The following example illustrates the invention in more details.

### EXAMPLE

| Soft vaginal capsules | |
|---|---|
| | Capsule composition |
| Lactobacillus fermentum | NLT 10¹⁰ (equal to about 1 g of lyophil) |
| Medium-chain triglicerides | 1964 mg |
| Silica (Aerosil 300) | 36 mg |

### Preparation:

The substance, obtained as described in EP-B-353 581, is mixed with Aerosil 300 under nitrogen current. The mixture is disperded in Migliol^{(R)} with slow stirring and always under nitrogen current, till an oily homogeneous suspension is obtained.

The obtained suspension is refined by colloidal mill to assure the homogeneous fragmentation of the particles in the mass, carefully controlling that the temperature thereof does not exceed 30°C.

The suspension is then subjected to vacuum activity by means of a proper equipment so that the air which eventually has been incorporated during the previous operations can be eliminated, in order to exclude any possible oxidation of the active ingredient and in order to guarantee a perfect volumetric dosage of the mixtures in the capsules.

The suspension is then incapsulated in glycogelatin envelopes (Softigel^{(R)}, Scherer) extruded by a proper encapsulator. The capsules, after drying for 60 hours at 20°C and with relative humidity lower than 45%, are distributed in glass flasks with cap-reservoir containing the silica gel.

## Claims

1. Soft gelatin vaginal capsules containing a therapeutically active dose of lyophilized Lactobacillus fermentum I-789 suspended in a suitable vehicle, **characterized in that** they contain from 2 to 5% by weight of silica gel or precipitated silica, with respect to the weight of the lyophilizate.

2. Capsules according to claim 1, wherein the vehicle is constituted by medium-chain triglycerides.

3. Capsules according to claim 1 or 2 wherein the silica is comprised between 3 and 4% by weight, with respect to the weight of the lyophilizate.

4. Capsules according to any of claims 1-3 wherein the gelatin-capsules are constituted by a mixture of gelatin, glycerol, dimethylpolysiloxane and titanium dioxide.

5. Capsules according to any of the previous claims containing other eubiotic bacteric strains in combination with Lactobacillus fermentum I-789.

## Patentansprüche

1. Vaginale Weichgelatinekapseln, enthaltend eine therapeutisch aktive Menge von gefriergetrocknetem *Lactobacillus fermentum* I-789 suspendiert in einem geeigneten Mittel, **dadurch gekennzeichnet, daß** sie zwischen 2 und 5% in Gewicht von Silikagel oder gefälltem Silika enthalten, bezogen auf das Gewicht des gefriergetrockneten Produkts.

2. Kapseln nach Anspruch 1, wobei das Mittel mittelkettige Triglyceride sind.

3. Kapseln nach Anspruch 1 oder 2, wobei das Silika zwischen 3 und 4% in Gewicht liegt, bezogen auf das Gewicht des gefriergetrockneten Produkts.

4. Kapseln nach einem der Ansprüche 1-3, wobei die Gelatinekapseln aus einer Mischung von Gelatine, Glyzerin, Dimethylpolysiloxan und Titandioxide bestehen.

5. Kapseln nach einem der vorstehenden Ansprüche, die andere eubiotike Bakterienstämme in Kombination mit *Lactobacillus fermentum* I-789 enthalten.

## Revendications

1. Capsules molles vaginales, contenant une quantité therapeutiquement efficace de *Lactobacillus fermentum* I-789 lyophilisé en suspension dans un véhicule approprié, **caractérisées en ce que** elles contiennent entre 2 et 5% en poids de gel de silice ou silice précipité, calculé sur le poids du lyophilisat.

2. Capsules selon la revendication 1, dans lesquelles le véhicule est constitué par des triglycérides à chaîne moyenne.

3. Capsules selon la revendication 1 ou 2, dans lesquelles le silice est compri entre 3 et 4% en poids, calculé sur le poids du lyophilisat.

4. Capsules selon l'une quelconque des revendications 1-3, dans lesquelles les capsules de gélatine sont constituées par un mélange de gélatine, glycérol, diméthylpolysiloxane et dioxide de titane.

5. Capsules selon l'une quelconque des revendications précédentes, contenant autres souches microbiennes eubiotiques en association avec *Lactobacillus fermentum* I-789.
